# EUROPÄISCHE PATENTSCHRIFT

(11) **EP 0 323 528 B1**
(45) Veröffentlichungstag und Bekanntmachung des Hinweises auf die Patenterteilung: **15.09.1993**
(21) Anmeldenummer: 88906226.1
(22) Anmeldetag: 26.05.1988
(51) Int. Cl.: A61B 17/28

(54) **Chirurgische Klemme**
Surgical Forceps
Pince Chirugicale

(30) Priorität: 26.06.1987 SU 4268325
(43) Veröffentlichungstag der Anmeldung: 12.07.1989
(73) Patentinhaber: VSESOJUZNY NAUCHNO-ISSLEDOVATELSKY I ISPYTATELNY INSTITUT MEDITSINSKOI TEKHNIKI, Moscow, 129301 (RU)
(72) Erfinder: AKOPOV, Ernest Mikhailovich, Moscow, 123631 (SU); KAPITANOVA, Elena Nikolaevna, Moscow, 123298 (SU)
(74) Vertreter: Nix, Frank Arnold, Dr.
(86) Internationale Anmeldenummer: SU8800123
(87) Internationale Veröffentlichungsnummer: WO8810097

(56) Entgegenhaltungen:
- US-A- 3 454 009
- US-A- 3 503 398
- AESCULAP-Katalog; "Instrumente für Diagnostik, kleine und grosse Chirurgie, Urologie, Gynäkologie und Geburtshilfe"; Band I, 9. Auflage

## Beschreibung

### Technisches Gebiet

Die Erfindung bezieht sich auf das Gebiet Medizintechnik, insbesondere auf chirurgische Klemmen zum Ergreifen und Halten von Geweben oder Organen während der verschiedenartigen Handhabungen in einer Operationswunde im Laufe von selbst unterschiedlichsten chirurgischen Operationen, besonders wenn die Höhle eines Organs einwandfrei zugeschlossen und dessen Exzisionsteil gehalten werden muß, das nach der Anlegung einer mechanischen Klammernaht vermittels eines Nähapparats beispielsweise bei der Magen-, Darmresektion oder bei der Resektion anderer Organe in der Bauch- und Brusthöhle abgeschnitten wird.

### Zugrundeliegender Stand der Technik

Bekannt sind chirurgische Klemmen zum Ergreifen und Zurückhalten von Geweben oder Organen, die zwei miteinander beweglich gekoppelte Klemmenhälften einschließen, welche langgestreckte Backen mit Druck- und Außenflächen an deren Arbeitsabschnitt aufweisen. An der Druckfläche des Arbeitsabschnitts der Backen werden diese Klemmen mit einer Einkerbung (s. beispielsweise den Katalog der Firma "Aesculap" "Instrumente für Diagnostik, kleine und große Chirurgie, Urologie, Gynäkologie und Geburtshilfe", Band 1,9. Auflage, S. 266, Nr. B-21178) oder mit längsverlegten Rillen und Vorsprüngen auf jeder Backe versehen, die derart angeordnet sind, daß die Vorsprünge einer Backe in die Rillen der anderen hineinragen (s. denselben Katalog, S. 267, Nr. B-21181 DF).

Die Einkerbung sowie die Rillen mit den Vorsprüngen sind zur Verstärkung der Haftkraft zwischen den Oberflächen der Gewebe oder Organe und der Druckfläche des Arbeitsabschnitts der Backen bestimmt. Trotzdem ermöglichen die erwähnten Klemmen keinerlei zuverlässiges Festhalten von Geweben oder Organen zwischen den Backen, ohne daß diese übermäßig zusammengedrückt werden, was Verletzungen verursacht. Die bekannten Klemmen geben keine Möglichkeit, das Herausgleiten der zusammengedrückten Wände der Organe zu verhindern, besonders wenn die Gewebe in Senkrechtrichtung in bezug auf die Backen der Klemmen aufgespannt werden. Das kommt beispielsweise bei dem Abschneiden des Exzisionsteils eines Organs unmittelbar an den Backen der Klemme zustande, nachdem bei der Organenresektion eine mechanische Klammernaht mit Hilfe eines chirurgischen Nähapparats angelegt ist. Durch das eventuelle Herausrutschen der Gewebe aus den Backen der Klemme während der verschiedenartigen Handhabungen im Laufe einer Operation tritt die Gefahr auf, die Operationswund zu infizieren, solange die Organhöhle geöffnet ist und die verschmutzte Innenfläche des betreffenden Organs mit den Umgebungsgeweben in Berührung tritt, aus der Höhle des erwähnten Organs der infizierte Inhalt herausfließen kann und die Hämostase beeinträchtigt wird. Zur Verstärkung der Haftkraft an der Druckfläche der Klemmenbacken mit den Geweben müssen die Backen bei den bekannten Klemmen mit einer größeren Breite ausgeführt werden, was mit Rücksicht auf die Operationsverhältnisse, beispielsweise beim Fehlen freier Gewebeteile ihre Anwendung nicht immer ermöglicht, welche Gewebeteile zum Anlegen der Klemmenbacken in ihrer Arbeitsstellung vorhanden sein müssen. Darüber hinaus muß auch die Starrheit der fliegenden Klemmenbacken erhöht werden, um die Wandung des jeweiligen Organs mit Sicherheit festzuhalten, da in mehreren Fällen die Zusammendruckkräfte an den Wänden der Organe, beispielsweise bei dem Zusammendrücken und Festhalten der Wände des Exzisionsteils des Magens während dessen Resektion ziemlich groß sein müssen, um das Herausrutschen der Gewebe zu vermeiden. Dadurch werden die Klemmen mit ihren breiten und erhöhten Backen äußerst sperrig, was das Manövriervermögen mit diesen innerhalb der Operationswunde beinträchtigt. Nach dem Zusammendrücken der Wandungen von innerhalb des Organismus nach der Operation zurückbleibenden Geweben und Organen mit den betreffenden Klemmen kann Nekrose infolge eines übermaßigen Zusammendrucks auftreten.

Eine weitere bekannte Klemme, beispielsweise von Price-Thomas,a (s. den Katalog der Firma "Aesculap" "Instrumente für Diagnostik, Kleine und große Chirurgie, Urologie, Gynäkologie und Geburtshilfe", Band 1, 9, Auflage, Nr. B-21182, S. 269) schließt zwei miteinander beweglich gekoppelte Klemmenhälfte ein, die langgestreckte Backen mit Druck- und Außenflachen besitzen. In der Mitte der Druckfläche des Arbeitsabschnittes einer der Klemmenbacken entlang sind durchgehende Bohrungen ausgebildet. In diese Bohrungen ragen mit diesen übereinstimmende , spitze , ebenfalls an der Mittellinie der Backe angeordnete Kegelzähne der anderen Backe hinein, wenn die Backen zusammengeschwenkt werden.

Diese Klemme von Price-Thomas,a ist im praktischen Gebrauch dadurch unbequem, daß der Arbeitsabschnitt beider Backen eine große Breite aufweist. Die Starrheit der Backen nimmt durch die Ausbildung der Durchgangsbohrungen wesentlich ab, welche Durchgangsbohrungen eine Spannungsanhäufung und den Verlust an Festigkeit in den Backen verursachen, besonders wenn diese eine große Länge aufweisen. Infolgedessen können die Backen während der Operation beim Zusammendrücken von massiven und dichten Organwände in Stücke gehen. Aus diesem Grunde müssen die Backen der Price-Thomas,a-Klemmen breit und kurz ausgeführt werden, wodurch keine Möglichkeit besteht, diese Klemmen zum Ergreifen und Festhalten von Geweben einer verhältnisßmäßig großen Langstreckung zu benutzen. Durch Anbringung der Kegelzähne nur auf einer Backe wird das sichere Festhalten ebenfalls nur einer Seite der zusammengedrückten Wandung eines Organs, beispielsweise des Magens oder Dickdarms erreicht. Die andere, an der Backe mit den Bohrungen liegende Wand kann herausrutschen, wodurch sich die Höhle des jeweiligen Organs öffnet und die Aseptik der Operation verloren geht. Um die Zuverlässigkeit des Ergreifens und Festhaltens der Gewebe zu steigern, muß die Höhe der Kegelzähne vergrößert werden, damit die beiden Wände eines dickwandigen Organs durchgestochen werden können. Jedoch macht diese Vergrößerung der Zahnhöhe die Kegelzähne weniger widerstandsfähig, bei den Handhabungen der Klemme unbequem, wobei sich die Wahrscheinlichkeit, die Operations- und Umgebungsgewebe, sowie die Hände des Chirurgen des öfteren zu verletzen, erhöht. Es muß auch darauf aufmerksam gemacht werden, daß die an der Mittellinie der Backen angeordneten Bohrungen kleineren Durchmessers Sammlungsstellen von Schmutz darstellen und bei deren Reinigung sehr unbequem sind.

Eine größere Zuverlässigkeit im Sinne des Ergreifens und Festhaltens beider Wände von Geweben und Organen bringt die bekannte Mikulicz-Klemme,a mit sich (s. denselben Katalog der Firma "Aesculap", S. 329, Nr. E-295). Zum Unterschied von der obenbeschriebenen Price-Thomas-Klemme trägt sie auf dem Arbeitsabschnitt jeder Backe an der Mittellinie entlangverlegte, über die Druckfläche hervortretende und der gegenüberliegenden Backe zugekehrte Zähne und besitzt zwischen den erwähnten Zähnen verteilte Durchgangsbohrungen, so daß die Zähne auf einer Backe gegenüber den Durchgangsbohrungen der gegenüberliegenden Backe stehen. Dadurch ermöglicht die Miculicz,a-Klemme ein zuverlässiges Festhalten beider Wände von Geweben oder Organen, die zwischen deren Backen zusammengedrückt werden. Jedoch muß auch bei dieser Klemme eine große Backenbreite in Kauf genommen werden, an den Backen werden keine ausreichenden Festigkeit und Starrheit erreicht, was auf die aufbaumäßigen Besonderheiten des Arbeitsabschnitts der Klemmenbacken zurückzuführen ist, welche die längs der Mittellinie der Backen angeordneten Zähne und Durchgangsbohrungen aufweisen. In diesem Zusammenhang kommt die Sperrigkeit der Klemme zustande, besonders wenn die Backen mit einer großen Länge ausgeführt werden, dazu müß eine große Breite der Gewebe vorhanden sein, die jeweils zur Aufstellung der Klemmenbacken in ihre Arbeitslage notwendig ist, während das Manövriervermögen beim Gebrauch der Klemme in der Operationswunde beeinträchtigt wird und das Brechen der Backen sowie die geringe Lebensdauer der betreffenden Klemmen auch die Folgen der erwähnten Aufbaubesonderheiten sind. Es muß noch die Unbequemlichkeit der Reinigung der an der Mittellinie entlang liegenden Durchgangsbohrungen und der Zähne in Kauf genommen werden.

Wegen der erwähnten Nachteile wird auch die Möglichkeit eingeschränkt, die bekannten Klemmen in der chirurgischen Praxis weitgehend anzuwenden, bei welcher die Wände von Organen auf einer verhältnismäßig großen Länge zuverlässig ergriffen und festgehalten werden müssen, wie dies beispielsweise bei dem Zusammendrücken des Exzisionsteils des jeweiligen Organs nach dem Anlegen einer mechanischen Klammernaht mit Hilfe eines Nähapparats der Fall ist. Die betreffenden bekannten Klemmen sind im Gebrauch unbequem oder überhaupt nicht anwendbar, wenn die Klemmenbacken wegen Mangels an freigegebenen Geweben nicht angelegt werden können. Diese Klemmen bringen bei ihren Handhabungen in einer tiefen und verengten Operationswunde, beispielsweise in der Proktologie bei der Arbeit im kleinen Becken, in der Thoraxchirurgie, bei den Operationen an Speiseröhren, in der Kinderchirurgie usw. Unbequemlichkeiten und traumatische Verletzungen mit sich.

### Offenbarung der Erfindung

Der vorliegenden Erfindung liegt die Aufgabe zugrunde, solch eine chirurgische Klemme zu entwickeln, bei der die aufbaumäßige Gestaltung und Anordnung der Einzelteile an der Druckfläche des Arbeitsabschnitts der Backen die Möglichkeit geben, das Manövriervermögen in der Operationswunde beim Gebrauch der Klemme erheblich zu steigern, die Lebensdauer der Klemme zu verlängern, die Verhältnisse ihrer Reinigung zu verbessern sowie die Zuverlässigkeit des Ergreifens und Festhaltens von Geweben und Wänden der Organe zu erreichen.

Die erwähnte Aufgabe wird dadurch gelöst, daß bei einer chirurgischen Klemme, die aus zwei miteinander beweglich gekoppelten Klemmenhälften besteht, deren jede mit einer langgestreckten Backe enden, die einen Arbeitsabschnitt mit einer Druckfläche aufweist, die mit abwechselnd angeordneten Zähnen und Ausnehmungen ausgestattet ist, welche mit den Ausnehmungen und Zähnen an der gegenüberliegenden Druckfläche der anderen Backe übereinstimmen, erfindungsgemäß die erwähnten Zähne und Ausnehmungen zumindest an einem Längsrand der Druckfläche angeordnet sind, wobei sich die Ausnehmungen in Richtung der Außenfläche des Arbeitsabschnitts der Backe öffnen.

Durch diese Ausführung der Klemme wird die Notwendigkeit vermieden, die festhaltenden Zähne und die mit diesen übereinstimmenden Durchgangsbohrungen an der Achse der Druckfläche entlang an dem Arbeitsabschnitt der Backen zu verteilen, wie sie bei den bekannten Klemmen verteilt werden, was eine wesentliche Verkleinerung der Breite des Arbeitsabschnitts der Backen, praktisch auf die Hälfte und mehr, unter Erhaltung eines gesicherten Ergreifens und Festhaltens von Geweben und Wandungen der Organe unterschiedlicher Dicke ermöglicht. Diese kleinere Backenbreite und demzufolge auch ein besseres Manövriervermögen beim Gebrauch der Klemme ermöglichen deren Benutzung an schwerzugänglichen Stellen und bei Mangel an den freigegebenen Geweben, an die die Klemme in der Arbeitsstellung angelegt werden muß, beispielsweise beim Zusammendrücken und Abschneiden an den Klemmenbacken des Exzisionsteils der Kehle mit dem Kehlkopf nach dem Anlegen der mechanischen Klammernaht an der Kehle mit Hilfe eines Nähapparats bei der Operation wegen des Kehlkopfkarzinoms. Durch die offene Gestaltung der Ausnehmungen in Richtung der Außenfläche des Arbeitsabschnitts der Backe und nicht in Form von durchgehenden bzw. blinden, Ansammlungsstellen von Schmutz darstellenden, wesentliche Spannungsanhäufungen bewirkenden Bohrungen, wie es bei den bekannten Klemmen der Fall ist, besteht die Möglichkeit, bei allen anderen gleichen Verhältnissen die Festigkeit der Backen zu steigern, deren Brechen zu vermeiden, die Verhältnisse der Reinigung nach der Operation zu verbessern und Wartungsdauer der Klemme zu verkürzen.

Zweckmäßigerweise ist ein Teil der Seitenfläche des Zahns als eine Verlängerung der Außenfläche des Arbeitsabschnitts der Backe auszuführen. Diese Vereinigung der erwähnten Oberflächen ermöglicht ein leichteres Saubermachen der Klemme und gestattet es, die Außenfläche des Arbeitsabschnitts der Backen ohne Vorsprünge auszuführen, was ebenfalls die Möglichkeit gibt, das Skalpell an dem Arbeitsabschnitt der Backen bei dem Zerschneiden der Gewebe, beispielsweise zwischen den die Wände des Exzisionsteils des Organs zusammendrückenden Klemmenbacken und dem Nähapparat nach dem Anlegen der mechanischen Klammernaht an das Resezierorgan bequem und zügig zu führen.

Es empfiehlt sich, daß die Oberfläche der Ausnehmung einen Teil der Mantelfläche eines Drehkörpers darstellt, deren im Axialschnitt der Ausnehmung liegende Erzeugende in bezug auf die Außenfläche des Arbeitsabschnitts der Backe schräg verläuft. Durch diese Ausführung der Klemme werden eine größtmögliche Festigkeit des Arbeitsabschnitts der Backen bei deren verhältnismäßig geringer Breite und ein erleichtertes Reinigen erzielt, weil die Ausnehmungen eine ununterbrochene, offene Oberfläche ohne Ecken, mit einem bequemen Zugang zu deren Reinigung sowohl von der Druck- als auch von der Außenfläche aus aufweisen.

Beim Vorhandensein der Zähne und Ausnehmungen an jedem Längsrand der Druckfläche des Arbeitsabschnitts der Backe entlang ist es zweckmäßig, die längs des einen Längsrandes ausgebildeten Zähne und Ausnehmungen gegenüber den längs des anderen Längsrandes ausgebildeten Zähne und Ausnehmungen zu versetzen. Diese Ausführung des Arbeitsabschnitts der Backen der Klemme ermöglicht ein gleichmäßiges Ergreifen und ein zuverlässigeres Festhalten der Gewebe und Wände von Organen längs des zusammengedrückten Streifens und wirkt sich auf die Festigkeit und Lebensdauer der Klemmenbacken durch die erwähnte Versetzung der Ausnehmungen an einem Längsrand der Druckfläche gegen die Ausnehmungen an dem anderen Längsrand günstig aus. Die Versetzung der Zähne an einem Längsrand der Druckfläche gegen die Zähne an dem anderen Längsrand bringt weitere, verbesserte Bequemlichkeiten beim Saubermachen der Druckfläche der Becken, weil dadurch ein besserer Zugang zum Zahngrund beim Reinigen erreicht und die Wahrscheinlichkeit des Zurückbleibens von Schmutz in diesem Bereich im Vergleich mit dem Fall herabgesetzt wird, bei dem die Zähne an den beiden Längsrändern der Druckfläche gegeneinander angeordnet werden.

Es ist vorteilhaft, die Breite des Arbeitsabschnitts wesentlich kleiner als die des Restteils der Backen auszuführen. Diese Möglichkeit besteht dank den oben erwähnten aufbaumäßigen Besonderheiten der Zähne und Ausnehmungen der erfindungsgemäßen Klemme, durch deren gegenseitige Anordnung und durch die Anordnung derselben bezüglich der Druckfläche und der Außenfläche des Arbeitsabschnitts der Backen, wodurch die zum Anlegen der Klemme in deren Zusammendrückstellung notwendige Gewebebreite wesentlich herabgesetzt und das Manövriervermögen bei Gebrauch der Klemme in der Operationswunde stark verbessert werden kann. In diesem Zusammenhang ermöglicht der Aufbau der Klemme, bei der die Breite des Arbeitsabschnitts wesentlich kleiner als der Restteil ist, an den Klemmen der erwähnten Bauart die in ihren Abmessungsreihen standardisierten Verschlußverbindungen auszunutzen, die im allgemeinen in Klemmen anderer Bauart ihre Anwendung finden, und zwar beispielsweise standardisierte Schachtelverschlüsse, was die Möglichkeit bietet, die Bauelemente der Klemmen unterschiedlicher Bauarten zu vereinheitlichen und demzufolge deren Fertigung zu vereinfachen.

Der Arbeitsabschnitt der Backen kann derart ausgeführt werden, daß dessen Höhe in Richtung von dem Ende des Arbeitsabschnitts aus zu dessen Grundfläche zunimmt, wobei diese Höhe des Arbeitsabschnitts der Backen an der Grundfläche größer als die Höhe des Restteils der Backen auszuführen ist. Beim Zusammendrücken von dichten und massiven Geweben und Organwänden mit großen Abmessungen über die Zusammendrucklänge wird dadurch eine erhöhte Starrheit des verjüngten Arbeitsabschnitts der Backen über ihre Breite erreicht, die Gewebe werden demzufolge zuverlässig ergriffen und festgehalten. Dabei werden die Kompaktheit des Restteils der Klemme und die Bequemlichkeit des Gebrauchs sowie ein verhältnismäßig geringes Gewicht der Klemme im Ganzen beibehalten.

Die Übergangsfläche zwischen dem schmaleren Arbeitsabschnitt und dem breiteren Restteil jeder Backe ist zweckmäßigerweise näher zu deren distalem Ende hin gelegen als die Übergangsfläche zwischen dem höheren proximalen Ende des Arbeitsabschnitts und dem niedrigeren Restteil jeder Backe. Bei einer verhältnismäßig geringeren Breite des Arbeitsabschnitts wird dadurch die erforderliche Starrheit der Klemmenbacken erzielt. Darüber hinaus sieht diese gegenseitige Anordnung der erwähnten Übergangsflächen in bezug auf den Verschlußteil ästhetisch besser aus.

Bei Klemmenhälften mit gekrümmten Backen, deren Arbeitsabschnitt unter einem Winkel zu der zur Gelenkachse senkrechten Ebene liegt, ist es zweckmäßig, die Übergangsflächen zwischen dem schmaleren bzw. dem am proximalen Ende höheren Arbeitsabschnitt der Backen und dem Restteil der Backen auf der Gelenkseite anzuordnen. Dadurch werden ein besseres Manövriervermögen der Klemme in der verengten Operationswunde und die Bequemlichkeit der Handhabungen beim Heranführen der Klemme an die Stelle des Zusammendrückens der Gewebe und Organe beispielsweise innerhalb des kleinen Beckens bei Operationen am Mastdarm oder bei Operationen an der Speiseröhre erreicht.

Durch die aufbaumäßigen Besonderheiten der erfindungsgemäßen Klemme werden eine Erweiterung ihrer Funktionsmöglichkeiten, eine erhöhte Zuverlässigkeit im Gebrauch und eine bessere Bequemlichkeit der Wartung der Klemme erzielt.

### Kurzbeschreibung der Zeichnungen

Nachstehend wird die Erfindung anhand der Beschreibung konkreter, sie aber nicht einschränkender Ausführungsbeispiele unter Bezugnahme auf die Zeichnungen näher erläutert; in diesen zeigt:
Fig. 1 Vorderansicht der erfindungsgemäßen chirurgischen Klemme in schematischer Darstellung;
Fig. 2 Seitenansicht der erfindungsgemäßen chirurgischen Klemme;
Fig. 3 Einzelteil des Arbeitsabschnitts der erfindungsgemässen Klemme im vergrößerten Maßstab;
Fig. 4 Arbeitsabschnitt der erfindungsgemäßen Backen der Klemme mit längs eines Längsrandes der Druckfläche der Backen ausgebildeten Zähnen und Ausnehmungen, in räumlicher Darstellung;
Fig. 5 Einzelheit des Arbeitsabschnitts der Backen der Klemme nach der Fig. 4 mit Zähnen und Ausnehmungen, im vergrößerten Maßstab;
Fig. 6 Einzelheit des Arbeitsabschnitts der Backen der erfindungsgemäßen Klemme mit längs beider Längsränder der Druckfläche der Backen ausgebildeten Zähnen und Ausnehmungen, in räumlicher Darstellung;
Fig. 7 Schnitt VII-VII der Fig. 3 mit längs eines Längsrandes der Druckfläche des Arbeitsabschnitts der Backen ausgebildeten Zähnen und Ausnehmungen;
Fig. 8 Schnitt VIII-VIII der Fig. 3 mit längs eines Längsrandes der Druckfläche des Arbeitsabschnitts der Backen ausgebildeten Zähnen und Ausnehmungen;
Fig. 9 Schnitt IX-IX der Fig. 3 mit längs beider Längsränder der Druckfläche des Arbeitsabschnitts der Backen ausgebildeten Zähnen und Ausnehmungen;
Fig. 10 Schnitt X-X dar Fig. 3 mit längs beider Längsränder der Druckfläche des Arbeitsabschnitts der Backen ausgebildeten Zähnen und Ausnehmungen;
Fig. 11 mit dem Arbeitsabschnitt der Backen der erfindungsgemäßen Klemme zusammengedrückte Wände eines Organs, geschnitten an der Außenfläche der Klemmenbacken;
Fig. 12 Schnitt XII-XII der Fig. 11;
Fig. 13 Schnitt XIII-XIII der Fig. 11;
Fig. 14 einen Teil der erfindungsgemäßen Klemme, in Seitenansicht;
Fig. 15 einen anderen Teil der erfindungsgemäßen Klemme, in Seitenansicht;
Fig. 16 einen Teil der erfindungsgemäßen Klemme, Vorderansicht der Ausführungsform nach Fig. 15;
Fig. 17 erfindungsgemäße chirurgische Klemme mit gekrümmten Backen, in Vorderansicht;
Fig. 18 erfindungsgemäße chirurgische Klemme mit gekrümmten Backen, in Seitenansicht;
Fig. 19 erfindungsgemäße chirurgische Klemme mit gekrümmten Backen, in Draufsicht;

### Bevorzugte Ausführungsvariante der Erfindung

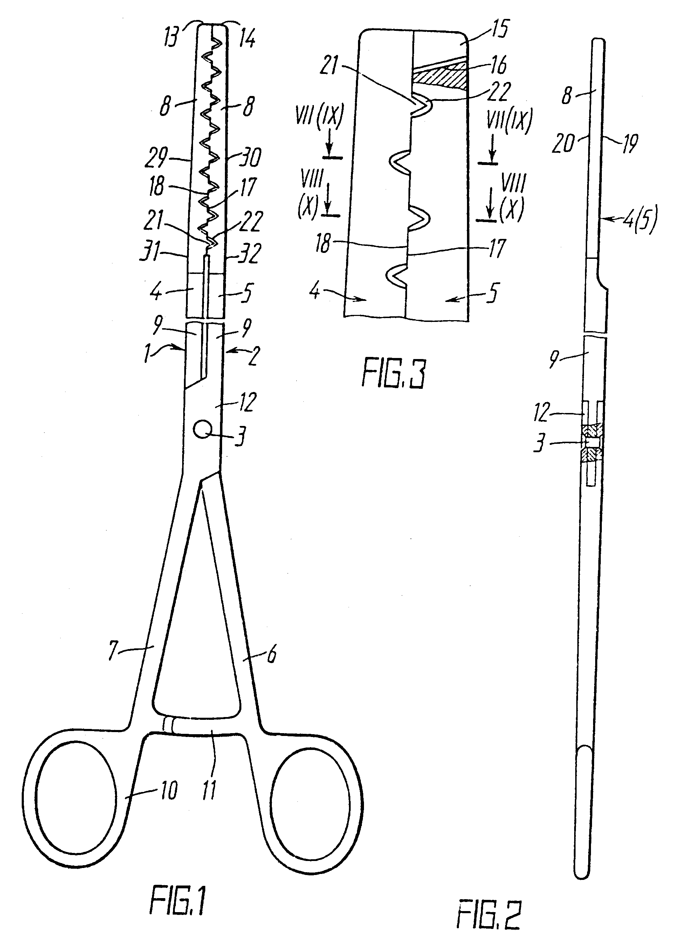

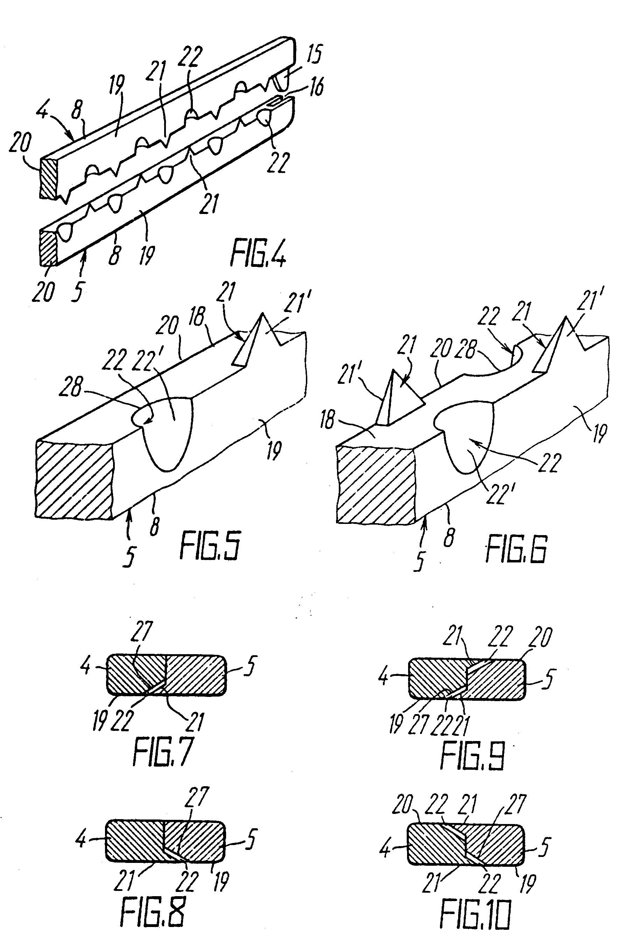

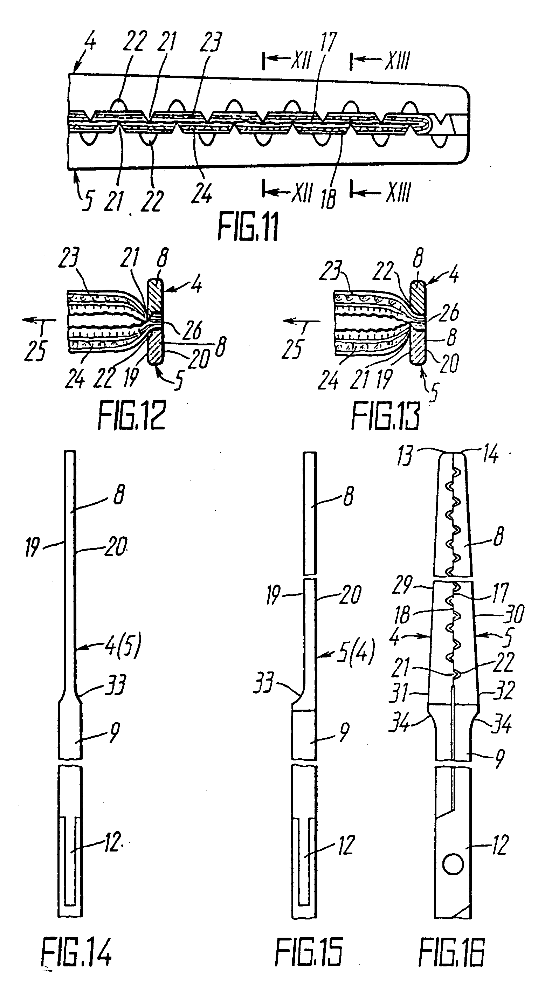

Die angemeldete chirurgische Klemme besteht aus zwei Hälften 1 und 2 (Fig. 1), die miteinander beweglich gekoppelt sind. In dem nachstehend beschriebenen, konkreten Ausführungsbeispiel kommt diese bewegliche Kopplung vermittels eines Gelenks 3 zustande, das eine gegenseitige Verschwenkung der Klemmenhälften 1, 2 ermöglicht. Außerdem können die Hälften 1, 2 der angemeldeten Klemme miteinander auch mit Hilfe von aus der Zeichnung nicht ersichtlichen Führungen gekoppelt werden, die eine parallele gegenseitige Verstellung der Klemmenhälften 1, 2 ermöglichen. Jede der Klemmenhälften 1,2 endet mit einer langgestreckten Backe 4 bzw. 5 und weist einen Handgriff 6, bzw. 7 auf. Die Backen 4 und 5 bestehen aus einem Arbeitsabschnitt 8 und einem zwischen diesem Arbeitsabschnitt 8 und dem Gelenk 3 befindlichen Restteil 9. Bei der in der Zeichnung wiedergegebenen Ausführung der Klemme sind die Handgriffe 6, 7 mit Osen 10 versehen und miteinander über einen Zahnstangenverschluß 11 verbunden, während die Klemmenhälften 1, 2 vermittels eines Schachtelverschlusses 12 miteinander in Verbindung stehen (Fig. 2). Die Enden 13, 14 (Fig. 1) des Arbeitsabschnitts der Klemmenbacken 4 bzw. 5 können an einer Backe, beispielsweise an der Backe 4 einen Vorsprung 15 (Fig. 3) haben, der in einen in der anderen, beispielsweise in der Backe 5 eingearbeiteten Schlitz 16 nineinragt, wodurch die Backen 4, 5 gegen gegenseitige Verstellungen in Querrichtung gesichert sind.

Die Backen 4, 5 (Fig. 1) weisen an ihren Arbeitdabschnitten 8 Druckflächen 17, 18 (Fig. 4) und Außenflächen 19, 20 auf. An der Druckfläche 17 bzw. 18 des Arbeitsabschnitts 8 jeder Backe sind abwechselnd Zähne 21 und Ausnehmungen 22 angeordnet. Die erwähnten Zähne 21 ragen über die Druckflächen 17, 18 heraus und sind mit ihren Spitzen der entgegengesetzten Backe zugewandt. Die Ausnehmungen 22 sind zwischen den Zähnen 21 derart verteilt, daß dadurch die Zähne 21 der Backe 4 genau gegen die Ausnehmungen 22 der Backe 5 und umgekehrt die Zähne 21 der Backe 5 genau gegen die Ausnehmungen 22 der Backe 4 stehen. Die Zähne 21 und die Ausnehmungen 22 können an einem Längsrand der Druckfläche 17, 18 des Arbeitsabschnitts 8 der Backen entlang, beispielsweise auf der Seite der Außenfläche 19 (Fig. 4, 5) angeordnet werden. Man kann sie auch an den beiden Längsrändern der Druckfläche, wie dies beispielsweise an der Druckfläche 18 veranschaulicht ist (Fig. 6), auf der Seite der beiden Außenflächen 19, 20 ausführen. Die Ausnehmungen sind dabei in Richtung der Außenfläche 19 bzw. 20 des Arbeitsabschnitts 8 der Backen, z. B. der Backe 5, geöffnet. Bei dem vollkommenen Zusammenschließen der Arbeitsabschnitte 8 der Backen 4 und 5 (Fig. 3, 7 bis 10) der Klemme treten die Zähne 21 in die Ausnehmungen 22 hinein.

Die bereits beschriebenen Besonderheiten der Klemmenausführung geben die Möglichkeit, den Arbeitsabschnitt 8 der Backen recht schmal auszuführen, praktisch um das 2fache und darüber kleiner als die Breite der bisher bekannten Klemmen, und gleichzeitig ein zuverlässiges Ergreifen sowie Festhalten der Wände 23, 24 (Fig. 11) des jeweiligen Organs zu gewährleisten. Unabhängig von der Stärke der Wände 23, 24 stechen die spitzen Zähne 21 zugleich in die beiden Wände 23, 24 des Organs bei deren Zusammendrücken zwischen den Backen 4, 5 hinein und verhindern deren Herausgleiten selbst bei einer starken Zugspannung der Gewebe in Richtung des Pfeiles 25 (Fig.12, 13). Die Gewebe werden auch in dem Fall zuverlässig festgehalten, wenn die zusammengedrückten Wände 23, 24 des Organs an den Backen(Gewebeschnitt 26) seitens der Außenfläche 20 abgeschnitten sind, die auf der den Zähnen 21 und Ausnehmungen 22, die bei deren einseitigen Anordnung auf der Seite der Außenfläche 19 liegen, gegenüberliegenden Seite befindlich ist. In diesem Zusammennang sind die erfindungsgemäßen Klemmen bei deren Anwendung samt den Nähapparaten zwecks Verschließens der Höhle und Festhaltens des Exzisionsteils von Organen wirksam, der nach dem Anlegen der mechanischen Naht vom Restteil der Organe abgeschnitten wird. Bei der zweiseitigen Anordnung dar Zähne 21 und der Ausnehmungen 22 (Fig. 5, 9, 10) wird die Verlegung des Gewebeschnitts 26 an dieser oder jeder Außenfläche 19 bzw. 20 des Arbeitsabschnitts 8 in Hinsicht der Erreichung eines zuverlässigen Ergreifens und Festhaltens der Wände des Organs gleichgültig. Der schmale Arbeitabschnitt 8 (Fig. 2) der Backen 4, 5 (Fig. 3) der Klemme ermöglicht deren gute Manövrierfähigkeit innerhalb der Operationswunde. Die Ausnehmungen 22 (Fig. 4, 5), die seitlich und an der Druckfläche der Backen geöffnet sind, können leicht bei der Reinigung der Klemme saubergemacht werden, da sie Blutgerinnsel und Schmutz nicht zurückhalten und gut durchgewaschen werden können.

Der Teil 21'der Seitenfläche der Zähne 21 (Fig. 5, 6) stellt eine Verlängerung der Außenfläche 19 (oder auch 20 bei der zweiseitigen Anordnung der Zähne 21 in der Fig.6) des Arbeitsabschnitts 8 (Fig. 1) der Backen 4, 5 dar. In dem konkreten Ausführungsbeispiel sind die Zähne 21 (Fig. 5, 6) in Form einer Pyramide mit einem spitzen Ende ausgeführt. Die Zähne 21 können auch in einer anderen, aufbaumäßig möglichen Ausführungsform, beispielsweise in Form eines aus der Zeichnung nicht ersichtlichen, Kegels gestaltet werden, der längs einer achsparallelen Ebene abgeschnitten ist. Die Oberfläche 22' der Ausnehmung 22 stellt einen Teil der Oberfläche eines Drehkörpers dar, deren im Axialschnitt der Ausnehmung 22 liegende Erzeugende 27 (Fig. 7 bis 10) in bezug auf die Außenfläche 19 bzw. 20 des Arbeitsabschnitts 8 der Backen schräg verläuft. Dies gestattet es, bei einer verhältnismäßig geringen Breite des Arbeitsabschnitts 8 (Fig. 1, 2) der Backen 4, 5 der Klemme deren Festigkeit zu steigern, die Spannungsanhäufung im Bereich der Ausnehmungen 22 (Fig. 5, 6) bei dem Zusammendrücken von Geweben und Wänden der Organe herabzusetzen und die Reinigung der Klemme zu erleichtern. Im Querschnitt stellt die Ausnehmung 22 einen Kreisbogen 28 dar. Die Zähne 21 und die Ausnehmungen 22 sind zweckmäßigerweise an dem Arbeitsabschnitt 8 der Backen entlang mit ein und derselben Teilung anzuordnen, wobei die Ausnehmungen 22 in einem Abstand von den benachbarten Zähnen 21 auszusparen sind, der der Teilungshälfte gleich ist.

Bei einer der möglichen Ausführungsformen der Klemme mit den an jedem Längsrand der Druckfläche 18 des Arbeitsabschnitts 8 der Backen angeordneten Zähnen 21 (Fig. 6) und Ausnehmungen 22 werden die bereits genannten Zähne 21 und Ausnehmungen 22 an einem Längsrand auf der Seite der Außenfläche 19 gegen die Zähne 21 und die Ausnehmungen 22 des anderen auf der Seite der Außenfläche 20 liegenden Längsrandes versetzt. Diese Versetzung der gleichnamigen Bauelemente, die längs beider Ränder der Druckfläche des Arbeitsabschnitts 8 der Backen ausgebildet sind, beträgt zweckmäßigerweise eine halbe Teilung, damit die Zähne 21 und die Ausnehmungen 22 in den beiden Reihen schachbrettartig angeordnet sind. Durch diese Ausführung des Arbeitsabschnitts 8 (Fig. 1) der Backen 4, 5 wird die Zuverlässigkeit des Ergreifens der Wände des Organs infolge einer größeren Dichte deren Einstiches mit den Zähnen 21 (Fig.6) vergrößert, wobei die Wände nicht auf einer Linie, sondern im Bereich der ganzen von der Breite des Arbeitsabschnitts 8 der Backen, beispielsweise der Backe 5 begrenzten Fläche durchgestochen werden. Durch die Versetzung der Zähne 21 an dem einen Längsrand der Druckfläche 18 des Arbeitsabschnitts 8 der Backe 5 gegen die Zähne 21 an dem anderen Längsrand wird die Druckfläche 18 der Backe 5 am Grund der Zähne 21 geöffnet, was sich bei der Reinigung der Klemme im Vergleich mit dem Fall günstiger auswirkt, bei dem diese Zähne gegeneinander angeordnet sind. Die entsprechende Längsversetzung der Ausnehmungen 22 in den beiden Reihen sieht im Sinne der Festigkeit und Starrheit des Arbeitsabschnitts 8 der Backen 5 bzw. 4 günstiger aus, was bei deren geringer Breite von Bedeutung ist.

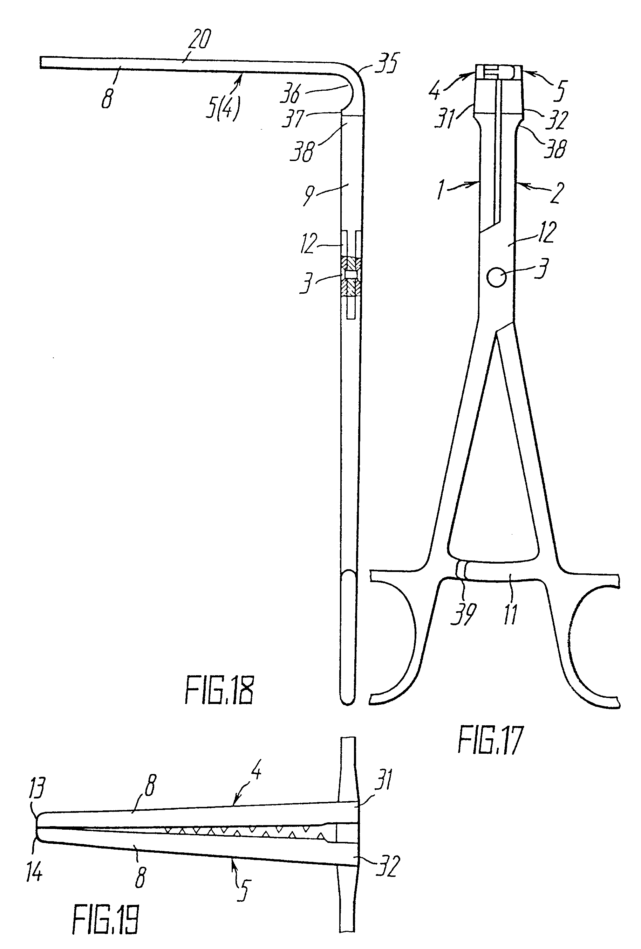

Die Breite des Arbeitsabschnitts 8 (Fig. 2, 14, 15) der Backen 4, 5 der Klemme ist wesentlich kleiner als die Breite des Restteils 9 ausgeführt. Die Außenflächen 19, 20 des Arbeitsabschnitts 8 der Backen 4, 5 verlaufen parallel zueinander, während die den Druckflächen 17, 18 gegenüberliegenden Oberflächen 29, 30 (Fig. 1,16) derart schräg verlaufen, daß die Höhe des Arbeitsabschnitts 8 der Backen 4, 5 in Richtung von den distalen Enden 13, 14 zu den proximalen Enden 31 bzw. 32 des Arbeitsabschnittes 8 zunimmt, wobei diese Enden etwa mit der Lage der von den Klemmenenden entferntesten Zähne 21 bzw. Ausnehmungen 22 zusammenfallen. Die Gesamtheit der beschriebenen Aufbaumerkmale ermöglicht es, gleiche Verhältnisse beim Ergreifen, Zusammendrücken und Festhalten der Gewebe und der Wände von Organen mit den Zähnen 21 über die ganze Länge des Arbeitsabschnitts 8 der Backen 4, 5 zu erhalten, der eine verhältnismäßig kleine Breite aufweist und demzufolge auch eines in seiner Breite schmalen Abschnitts von Geweben zur Anlegung des Arbeitsabschnitts 8 der Backen 4, 5 bedarf. Dabei werden auch die notwendige Starrheit des langgestreckten Restteils 9 der Backen und die Möglichkeit gesichert, den Verschluß 12 und die übrigen Einzelteile der Klemme, wie Handgriffe 6,7, Zahnstangenverschluß 11 und andere Bauelemente in Form und Abmessungen für die Klemmen verschiedenartiger Bauarten mit unterschiedlichen Abmessungen des Arbeitsabschnitts der Backen in standardisierter und vereinheitlichter Ausführung auszunutzen.

Um die erforderliche Starrheit des Arbeitsabschnittes 8 (Fig. 15, 16) der Backen 4, 5 mit einer verhältnismäßig grossen Länge zu erreichen, die zum Zusammendrücken der recht dichten Gewebe und Wände von Organen mit einer verhältnismäßig großen Langstreckung vorgesehen sind (beim Zusammendrücken und Verschließen der Höhle des Exzisionsteils des Magens kann beispielsweise die Länge der Klammernahtlinie nach dessen Einnähen vermittels der mechanischen Klammernaht mit Hilfe eines Nähapparats 100 mm und darüber erreichen), wird die Höhe des Arbeitsabschnitts 8 der Backen 4, 5 an deren proximalen Ende 31, 32 (Fig. 16) größer als die des Restteils 9 ausgeführt. Durch diese Ausführung wird die Kompaktheit des Restteils der Klemme auch bei gegenüber der Breite des Restteils 9 kleinerer Breite des Arbeitsabschhitts 8 der Backen 4, 5 erhalten, die Bequemlichkeit beim Gebrauch gewährleistet und ein verhältnismäßig geringes Gewicht der Klemme im Ganzen gesichert sowie die Vereinheitlichung und Standardisierung der Bauelemente der Klemmen ermöglicht. Die Übergangsfläche 33 (Fig. 15) zwischen dem schmaleren Arbeitsabschnitt 8 der Backen 4, 5 und dem Restteil 9 liegt dabei den distalen Enden 13, 14 des Arbeitsabschnitts 8 näher als die Übergangsfläche 34 (Fig. 16) zwischen dem an den proximalen Enden 31, 32 höheren Arbeitsabschnitt 8 und dem Restteil 9 der Backen 4, 5. Diese Übergangsflächen 33, 34 sind in dem beschriebenen Ausführungsbeispiel der Klemme als Zylinderflächen ausgeführt.

Die erfindungsgemäße chirurgische Klemme kann gerade und auch gekrümmte Backen besitzen.

In einer der Ausführungsvarianten der erfindungsgemäßen Klemme (Fig. 17) weisen die Backen 4, 5 eine Krümmung auf. Daher steht der Arbeitsabschnitt 8 (Fig. 18) der Backen unter einem, beispielsweise rechten (90^{o}) Winkel zu der zur Achse des Gelenks 3 senkrechten Ebene. Die in Bezug auf das Gelenk 3 an der Außenseite befindliche Außenfläche 20 des Arbeitsabschnitts 8 der Klemmenbacken ist an der Krümmungsstelle mit einem zügigen Übergang 35 in Richtung des Restteils 9 der Backen 4, 5 versehen, wobei die Zylinderübergangsfläche 36 zwischen dem schmaleren Arbeitsabschnitt 8 und dem Restteil 9 mit einem Vorsprung 37 an der Innenseite liegt. Die Übergangsflächen 36 und 38 von dem schmaleren bzw. dem am proximalen Ende höherem Arbeitsabschnitt 8 der Backen 4, 5 zu dem Restteil 9 befinden sich auf der Seite des Gelenks 3. Diese Klemmenausführung mit den gekrümmten Backen ohne eckige Vorsprünge verleiht dem Arbeitsabschnitt 8 der Backen 4, 5 seine Kompaktheit mit einem guten Manövriervermögen in der verengten Operationswunde und den Backen im Ganzen ihre notwendige Starrheit. Um die elastischen Verformungen der fliegenden Backen 4, 5 (Fig. 19) der Klemme auszugleichen und ein über die ganze Länge des Arbeitsabschnitts 8 der Backen 4, 5 gleichmäßiges Zusammendrücken von Geweben zu erreichen, werden die Backen derart ausgeführt, daß in der Ausgangsstellung, bei der die Zähne 39 (Fig. 17) des Zahnstangenverschlusses 11 der Klemme noch vor deren gegenseitigem Ineingrifftreten einander berühren, die Enden 13, 14 (Fig.19) des Arbeitsabschnitts 8 der Backen 4, 5 ebenfalls in Berührung treten, während an der Grundseite 31, 32 die Backen noch in einem gewissen Abstand voneinander abstehen. Während des Zusammendrückens der Gewebe und Wände von Organen vermindert sich der Keilwinkel zwischen den Backen 4, 5 bis auf Null.

Die obenerwähnten Fig. 1 bis 9 veranschaulichen die Ausführungsvarianten der Klemme, bei denen die beiden Backen 4, 5 (Fig. 1, 3, 4, 11, 16) den Arbeitsabschnitt 8 mit den Zähnen 21 und Ausnehmungen 22 einschließen. Es ist jedoch selbstverständlich, daß bei sonstigen Ausführungen die erfindungsgemäße Klemme so gestaltet werden kann, daß sie nur auf einer Backe, beispielsweise auf der Backe 4 die Zähne 21 tragt, während die entsprechenden Ausnehmungen 22 nur auf der gegenüberliegenden Backe, beispielsweise auf der Backe 5 vorhanden sind, was aber in den Zeichnungen nicht wiedergegeben ist. Darüber hinaus kann der Arbeitsabschnitt 8 mit den Zähnen 21 und den Ausnehmungen 22 nicht nur geradlinig, sondern noch gekrümmt beispielsweise entlang eines Bogens ausgeführt werden.

Die in der Beschreibung sowie in den Zeichnungen wiedergegebenen Ausführungsvarianten der chirurgischen Klemme erschöpfen nicht alle möglichen, erfindungsgemäßen Versionen, die in der chirurgischen Praxis zweckmäßigerweise auftreten können.

Die erfindungsgemäße chirurgische Klemme wird folgendermaßen gebraucht:

Die Gewebe oder Wände 23, 24 (Fig. 11 bis 13) des jeweiligen Organs werden atraumatisch, wenn diese Gewebeabschnitte nach der Operation zurückbleiben müssen, oder fest zusammengedrückt, ohne daß dabei die Möglichkeit der Verletzung der Gewebe in Kauf genommen wird, wenn die Klemme an den Exzisionsteil des jeweiligen Organs angelegt wird. Unabhängig von der Stärke der zusammenzudrückenden Wände 23, 24 des Organs dringen die Zähne 21 (Fig.11) des Arbeitsabschnitts 8 der Backen 4, 5 in diese Wände 23, 24 hinein, indem sie diese Wände gegen das Herausgleiten aus dem Zwischenraum zwischen den Druckflächen 17, 18 (Fig. 11) der Klemmenbacken 4, 5 zuverlässig festhalten.

Wenn man zum Ergreifen und Festhalten der Gewebe oder Wände 23, 24 des jeweiligen Organs eine Klemme mit nur einer Reihe der Zähne 21 (Fig. 4, 5) und Ausnehmungen 22 verwendet, die sich auf der Seite nur einer der Außenflächen, beispielsweise der Außenfläche 19 des Arbeitsabschnitts 8 der Backen 4, 5 befinden, und im Laufe der Operation die Gewebe an den Klemmenbacken abgeschnitten werden müssen, muß die Klemme derart angelegt werden, daß die Schnittfläche des vorgesehenen Schnitts 26 (Fig. 12, 13) auf der Seite verläuft, die der Außenfläche 19 des Arbeitsabschnitts 8 der Backen 4, 5 gegenüberliegt, welche Außenfläche 19 an die Zähne 21 und Ausnehmungen 22 angrenzt.

Bei der Anwendung der Klemme mit zwei Reihen der Zähne 21 (Fig. 6) und Ausnehmungen 22 auf der Seite jeder Außenfläche 19 und 20 des Arbeitsabschnitts 8 der Backen 4, 5 der Klemme spielt zum Ergreifen und Festhalten der Gewebe oder Wände 23, 24 von Organen die Anordnung der Klemmenbacken 4, 5 in bezug auf die Schnittfläche 26 keine Rolle, wenn die Notwendigkeit auftritt, die Gewebe an den Klemmenbacken entlang abzuschneiden.

Bei Benutzung der erfindungsgemäßen Klemme samt einem Nähapparat zum Zusammendrücken und Festhalten des Exzisionsteils des Organs wird dieser Exzisionsteil (aus der Zeichnung nicht ersichtlich) nach dem Anlegen der mechanischen Klammernaht (beispielsweise an den Magen bei dessen Resektion) mit den Klemmenbacken ergriffen, die dann bis auf die Berührung mit den Backen des Nähapparats verschoben werden, wonach die Wande des jeweiligen Organs auf der Seite des Exzisionsteils zusammengedrückt werden. Durch die geringe Breite des Arbeitsabschnitts 8 (Fig. 2, 14, 15, 18) der Backen 4, 5 werden ein gutes Manövriervermögen der erfindungsgemäßen Klemme bei deren Handhabungen und ein leichtes Heranführen der Klemme an die Stelle der Anlegung der mechanischen Naht erreicht. Hierbei werden die Zähne 21 (Fig. 12, 13) und die Ausnehmungen 22 der Klemme auf der der Anlegungssteile des Nähapparats gegenüberliegenden Seite angeordnet. Jetzt werden die Gewebe vermittels eines Skalpells zwischen den Klemmenbacken 4, 5 und den Nähapparat zerschnitten. Hiernach werden das Klammermagazin und die Matrize des Nähapparats auseinandergeführt, indem dadurch das mit der mechanischen Naht eingenähte Organ freigegeben wird. Dabei verschließen die Klemmenbacken 4, 5 die Höhle das Exzisionsteils des Organs mit Sicherheit, weil die Zähne 21 bes Arbeitsabschnitts 8 die Wände 23, 24 des jeweiligen Organs zuverlässig ergriffen haben und gegen das Herausgleiten fest zurückhalten, obwohl der Arbeitsabschnitt 8 der Klemmenbacken 4, 5 eine verhältnismäßig geringe Breite aufweist.

### Gewerbliche Verwertbarkeit

Auf diese Weise geben die aufbaumäßigen Besonderheiten der erfindungsgemäßen chirurgischen Klemme die Möglichkeit, ein gutes Manövriervermögen beim Gebrauch der Klemme unter den verschiedensten Anwendungsverhältnissen während der Operationen mit einer hohen Zuverlässigkeit des Ergreifens und Festhaltens der Gewebe und Wände von Organen, mit der Festigkeit der Backen und verlängerten Lebensdauer der Klemme sowie mit der Bequemlichkeit deren Wartung zu vereinigen. Diese Vorteile ermöglichen eine erfolgreiche Anwendung der erfindungsgemäßen Klemme, zum Beispiel der Klemme mit den gekrümmten Backen bei der Arbeit in der Tiefe einer verengten Operationswunde, beispielsweise bei den Operationen an der Speiseröhre oder in der Proktologie, wenn bei einem schweren Zugang eine einwandfreie Verschließung der infizierten Höhle des Endes des Mastdarms oder des Dickdarms nach dem Abschneiden des Resezierteils an den Klemmenbacken entlang und ein nachfolgendes Festhalten des Darmendes mit der verschlossenen Höhle bei den verschiedenartigen Handhabungen vorgenommen werden müssen, die zur weiteren Führung der chirurgischen Operation, beispielsweise zum Anlegen der Zwischendarmanastomose notwendig sind. Durch die genannten Vorteile ist die erfindungsgemäße Klemme auch in den Fällen anwendbar, wenn ein Mangel an zum Anlegen der Klemmenbacken an der Stelle des Ergreifens und Festhaltens der Gewebe notwendigen, freigegebenen Geweben in Kauf genommen werden muß. Durch diese Vorteile können die verschiedenen Ausführungsvarianten der Klemme in Verbindung mit Nähapparaten zum Zusammendrücken des Exzisionsteils des jeweiligen Organs nach dem Anlegen der mechanischen Klammernaht erfolgreich ausgenutzt werden.

Die Gesamtheit aller genannten Vorteile macht es möglich, die Qualität der Operationen zu steigern, die Arbeit des Chirurgen und der Bedienkraft zu vereinfachen, die Funktionsfähigkeiten der Klemmen der in Betracht genommenen Bauart auf verschiedenen Gebieten der Chirurgie zu erweitern.

## Patentansprüche

1. Chirurgische Klemme, die aus zwei miteinander beweglich gekoppelten Klemmenhälften (1, 2) besteht, deren jede mit einer langgestreckten Backe (4, 5) endet, die einen Arbeitsabschnitt (8) mit einer Druckfläche (17, 18) aufweist, welche mit abwechselnd angeordneten Zähnen (21) und Ausnehmungen (22) versehen ist, die mit den Ausnehmungen (22) und den Zähnen (21) an der gegenüberliegenden Druckfläche der anderen Backe übereinstimmen,
dadurch gekennzeichnet, daß die Zähne (21) und die Ausnehmungen (22) zumindest an einem Längsrand der Druckfläche (17, 18) angeordnet sind, wobei die Ausnehmungen (22) in Richtung der Außenfläche (19 oder 20) des Arbeitsabschnitts (8) der Backe (4, 5) offen sind.

2. Chirurgische Klemme nach Anspruch 1, dadurch gekennzeichnet, daß ein Teil (21') der Seitenfläche des Zahns (21) eine Verlängerung der Außenfläche (19 oder 20) des Arbeitsabschnitts (8) der Backe (4, 5) darstellt.

3. Chirurgische Klemme nach Ansprüchen 1, 2, dadurch gekennzeichnet, daß die Oberfläche (22') der Ausnehmung (22) einen Teil der Mantelfläche eines Drehkörpers darstellt, deren in Axialschnitt der Ausnehmung (22) liegende Erzeugende gegen die Außenfläche (19 oder 20) des Arbeitsabschnitts (8) schräg verläuft.

4. Chirurgische Klemme nach Ansprüchen 1 bis 3, dadurch gekennzeichnet, daß Zähne (21) und Ausnehmungen (22) längs beider Ränder der Druckfläche (17, 18) jedes Arbeitsabschnitts 8 ausgebildet sind und die Zähne und Ausnehmungen des einen Randes gegenüber den Zähnen und Ausnehmungen des anderen Randes versetzt sind.

5. Chirurgische Klemme nach Ansprüchen 1, 2, 3, 4, dadurch gekennzeichnet, daß die Breite des Arbeitsabschnitts (8) der Backen (4, 5) wesentlich kleiner als die Breite des Restteils (9) der Backen (4, 5) ausgeführt ist.

6. Chirurgische Klemme nach Anspruch 5, dadurch gekennzeichnet, daß die Höhe des Arbeitsabschnitts (8) der Backen (4, 5) in Richtung von den Enden (13, 14) des Arbeitsabschnitts (8) aus zu dessen Grundfläche (31, 32) zunimmt, wobei an dieser Grundfläche (31, 32) die Höhe des Arbeitsabschnitts (8) der Backen (4, 5) die des Restteils (9) der Backen übertrifft.

7. Chirurgische Klemme nach Ansprüchen 5 und 6, dadurch gekennzeichnet, daß die Übergangsfläche (33) zwischen dem schmaleren Arbeitsabschnitt (8) und dem breiteren Restteil (9) jeder Backe (4, 5) näher zu deren distalem Ende (13, 14) hin gelegen ist als die Übergangsfläche (34) zwischen dem höheren proximalen Ende (31, 32) des Arbeitsabschnitts (8) und dem niedrigeren Restteil (9) jeder Backe (4, 5).

8. Chirurgische Klemme nach Anspruch 7, dadurch gekennzeichnet, daß bei der Gelenkverbindung der Klemmenhälften (1, 2) mit gekrümmten Backen (4, 5), deren Arbeitsabschnitt (8) unter einem Winkel zu der der Gelenkachse (3) senkrechten Ebene verlegt ist, die Übergangsfläche (36) von dem verengten und die Übergangsfläche (38) von dem an der Grundfläche (31, 32) höheren Arbeitsabschnitt (8) der Backen (4, 5) zu deren Restteil (9) auf der Seite des Gelenks (3) angeordnet sind.

## Claims

1. Surgical clamp forceps which are composed of two forceps halves (1, 2) movably coupled to one another and each ending with an elongated jaw (4, 5) which has a working portion (8) with a pressure surface (17, 18) provided with alternately arranged teeth (21) and recesses (22) which match the recesses (22) and the teeth (21) on the opposite pressure surface of the other jaw, characterised in that the teeth (21) and the recesses (22) are arranged at least on one longitudinal edge of the pressure surface (17, 18), the recesses (22) being open in the direction of the outer surface (19 or 20) of the working portion (8) of the jaw (4, 5).

2. Surgical clamp forceps in accordance with Claim 1, characterised in that a part (21') of the lateral surface of the tooth (21) constitutes an extension of the outer surface (19 or 20) of the working portion (8) of the jaw (4, 5).

3. Surgical clamp forceps in accordance with Claims 1, 2, characterised in that the surface (22') of the recess (22) constitutes part of the generated surface of a body of revolution, the generatrix - located in the axial section of the recess (22) - of this generated surface running at an incline to the outer surface (19 or 20) of the working portion (8).

4. Surgical clamp forceps in accordance with Claims 1 to 3, characterised in that the teeth (21) and recesses (22) are formed along both edges of the pressure surface (17, 18) of each working portion (8) and the teeth and recesses of the one edge are staggered in relation to the teeth and recesses of the other edge.

5. Surgical clamp forceps in accordance with Claims 1, 2, 3, 4, characterised in that the width of the working portion (8) of the jaws (4, 5) is substantially less than the width of the remaining part (9) of the jaws (4, 5).

6. Surgical clamp forceps in accordance with Claim 5, characterised in that the height of the working portion (8) of the jaws (4, 5) increases from the ends (13, 14) of the working portion (8) to the base (31, 32) of the latter, at this base (31, 32) the height of the working portion (8) of the jaws (4, 5) exceeding that of the remaining part (9) of the jaws.

7. Surgical clamp forceps in accordance with Claims 5 and 6, characterised in that the transition region (33) between the narrower working portion (8) and the wider remaining part (9) of each jaw (4, 5) is located closer to the distal end (13, 14) of the latter than the transition region (34) between the higher proximal end (31, 32) of the working portion (8) and the lower remaining part (9) of each jaw (4, 5).

8. Surgical clamp forceps in accordance with Claim 7, characterised in that with the articulation of the forceps halves (1, 2) having curved jaws (4, 5) whose working portion (8) is at an angle to the plane perpendicular to the articulation axis (3), the transition region (36) from the thinner working portion (8) of the jaws (4, 5) to the remaining part (9) of the jaws and the transition region (38) from the working portion (8) of the jaws higher at the base (31, 32) to the remaining part (9) of the jaws are arranged on the same side as the articulation (3).

## Revendications

1. Pince chirurgicale composée de deux moitiés de pinces (1, 2) couplées cinétiquement l'une à l'autre dont chacune se termine par une tête allongée (4, 5) présentant une partie opérante (8) avec une surface de pression (17, 18) comportant des dents (21) et des évidements (22) disposés en ordonnance et qui correspond avec des évidements (22) et des dents (21) sur la surface de pression opposée de l'autre tête, caractérisée en ce que les dents (21) et évidements (22) sont disposés sur au moins un bord longitudinal des surfaces de pression (17, 18), les évidements (22) étant ouverts dans la direction des surfaces externes (19 ou 20) de la partie opérante (8) des têtes (4, 5).

2. Pince chirurgicale selon la revendication 1, caractérisée en ce qu'une partie (21') de la face latérale des dents (21) constitue un prolongement de la surface externe (19 ou 20) de la partie opérante (8) des têtes (4, 5).

3. Pince chirurgicale selon les revendications 1, 2, caractérisée en ce que la surface (22') de l'évidement (22) constitue une partie de la surface enveloppe d'un corps de rotation dont la génératrice disposée dans la section axiale de l'évidement (22) se présente obliquement contre la surface extérieure (19 ou 20) de la partie opérante (8).

4. Pince chirurgicale selon les revendications 1 à 3, caractérisée en ce que des dents (21) et des évidements (22) sont formés le long des deux bords de la surface de pression (17, 18) de chaque partie opérante (8) et les dents et les évidements de l'un des bords sont disposés de façon décalée par rapport aux dents et aux évidements de l'autre bord.

5. Pince chirurgicale selon les revendications 1, 2, 3, 4, caractérisée en ce que la largeur de la partie opérante (8) des têtes (4, 5) est essentiellement inférieure à la largeur de la partie restante (9) des têtes (4, 5).

6. Pince chirurgicale selon la revendication 5, caractérisée en ce que la hauteur de la partie opérante (8) des têtes (4, 5) augmente dans la direction partant depuis les extrémités (13, 14) de la partie opérante (8) jusqu'à la surface de base (31, 32) de celle-ci, de sorte que sur cette surface de base (31, 32), la hauteur de la partie opérante (8) des têtes (4, 5) dépasse celle de la partie restante (9) des têtes.

7. Pince chirurgicale selon les revendications 5 et 6, caractérisée en ce que la surface de transition (33) entre la partie opérante (8) plus étroite et la partie restante (9) plus large de chaque tête (4, 5) est plus proche à son extrémité distale (13, 14) que la surface de transition (34) entre l'extrémité proximale (31, 32) plus haute de la partie opérante (8) et la partie restante (9) plus basse de chaque tête (4, 5).

8. Pince chirurgicale selon la revendication 7, caractérisée en ce que, dans le cas de l'articulation des moitiés (1, 2) de la pince avec des têtes (4, 5) incurvées, dont la partie opérante (8) est disposée selon un angle par rapport à un plan perpendiculaire à l'axe d'articulation (3), la surface de transition (36) à partir de la partie opérante réduite et la surface de transition (38) à partir de la partie opérante (8) plus haute que la surface de base (31, 32) des têtes (4, 5) et la partie restante (9) sont disposées du côté de l'articulation (3).
